# EUROPÄISCHE PATENTSCHRIFT

(11) **EP 1 584 348 B1**
(45) Veröffentlichungstag und Bekanntmachung des Hinweises auf die Patenterteilung: **26.09.2007**
(21) Anmeldenummer: 04008242.2
(22) Anmeldetag: 05.04.2004
(51) Int. Cl.: A61M 39/10, A61B 5/022, F16L 37/084, F16L 37/02

(54) **Schlauchverbinder zum Verbinden von Messschläuchen für medizinische Überwachungseinrichtungen**
Tube connector for connecting measuring probes for medical surveillance devices
Raccord de tubes pour la connection de sondes de mesure utilisées dans des dispositifs médicaux de surveillance

(43) Veröffentlichungstag der Anmeldung: 12.10.2005
(73) Patentinhaber: Eckert, Dieter, 29640 Schneverdingen (DE); Quitsch, Peter, 22850 Norderstedt (DE)
(72) Erfinder: Eckert, Dieter, 29640 Schneverdingen (DE); Quitsch, Peter, 22850 Norderstedt (DE)
(74) Vertreter: Glawe, Delfs, Moll

(56) Entgegenhaltungen:
- EP-A- 0 240 452
- EP-A- 0 618 393
- EP-A- 1 355 099
- NL-A- 9 201 020
- US-A- 3 640 552
- US-A- 4 501 280
- US-A- 4 580 816
- US-A- 6 162 181
- US-A1- 2002 117 849

## Beschreibung

Die Erfindung betrifft Schlauchverbinder zum Verbinden von Messschläuchen für medizinische Überwachungseinrichtungen.

In Krankenhäusern müssen bspw. auf Intensivstationen oder während chirurgischer Eingriffe eine Vielzahl von Körperfunktionen von Patienten regelmäßig oder dauerhaft überwacht werden. Die Überwachung des Blutdruckes erfolgt mit Hilfe automatisierter Messgeräte. Die dem Patienten angelegte Messeinrichtung (z.B. Messmanschette) und das Messgerät müssen über eine Schlauchverbindung miteinander verbunden werden. Da das Messgerät und die Manschette in der Praxis trennbar sein müssen, weist eine Blutdruckmessmanschette üblicherweise einen kurzen Anschlussschlauch auf, der mittels einem angebrachten Schlauchverbinder mit dem am Messgerät angeschlossenen Schlauch verbunden wird.

Schlauchverbinder sind aus dem Stand der Technik bekannt.

EP-A-1 355 099 offenbart einen druckdichten und lösbaren Schlauchverbinder für flüssigkeitsführende Schlauchleitungen, beispielsweise Scheibenwaschflüssigkeitsschläuche in Kraftfahrzeugen.

Aus NL-A-9 201 020 ist ein Schlauchverbinder für ein Beatmungssystem eines Patienten bekannt. Der Schlauchverbinder ist so konstruiert, dass er erst beim Überschreiten einer gewissen Zugkraft an einer gewissen Stelle unterbrochen wird.

Gegenstand der US 3,640,552 sind Kupplungen zum Verbinden von Schläuchen, bei denen zwischen den beiden Kupplungsteilen eine Verkupplung über sich aus dem Außenbereich des einen Kupplungsteils erstreckende Einrastnasen, die in eine Einbuchtung an der Außenseite des zweiten Kupplungsteils von außen eingreift, sichergestellt wird.

Der Erfindung liegt die Aufgabe zu Grunde, einen einfach handhabbaren und im Krankenhausbereich vorteilhaft verwendbaren Schlauchverbinder der eingangs genannten Art zu schaffen.

Der erfindungsgemäße Schlauchverbinder weist zwei Kupplungsglieder mit folgenden Merkmalen auf:
a) die Kupplungsglieder können gasdicht und wieder lösbar zusammengesteckt werden,
b) jedes Kupplungsglied weist wenigstens einen Anschluss für einen Messschlauch auf,
c) die Kupplungsglieder sind im Kupplungsbereich einstückig aus einem Kunststoff geformt.
wobei das erste Kupplungsglied im Kupplungsbereich einen der Kupplungsseite zugewandten im wesentlichen als Hohlzylinder ausgebildeten Eintrittsabschnitt und axial daran anschließend einen gegenüber dem Eintrittsabschnitt radial nach außen erweiterten und eine größere radiale lichte Weite aufweisenden Halteabschnitt aufweist, dessen gegenüber dem Eintrittsabschnitt radial nach außen erweiterten Bereiche abgerundet ausgebildet sind; und dass das zweite Kupplungsglied kupplungsseitig einen Halteabschnitt aufweist, der im wesentlichen formschlüssig in den Halteabschnitt des ersten Kupplungsgliedes eingreifen kann und der gegenüber dem Eintrittsabschnitt des ersten Kupplungsgliedes ein radiales Übermaß aufweist, so dass er beim Zusammenstecken und Lösen der Kupplungsglieder unter elastischer Verformung eines oder beider Kupplungsglieder durch den Eintrittsabschnitt hindurchtreten kann, wobei im zusammengesteckten Zustand der Kupplungsbereich des zweiten Kupplungsgliedes radial vom Kupplungsbereich des ersten Kupplungsgliedes umgriffen wird, wobei die Kupplungsglieder im Kupplungsbereich durch elastischen Formschluss ineinandergreifen und die gegenüber dem Eintrittsabschnitt radial nach außen erweiterten Bereiche des Halteabschnitts gegenüber dem Halteabschnitt des zweiten Kupplungsgliedes eine Halte- und Dichtfunktion ausübt.

Zunächst seien einige im Rahmen der Erfindung verwendete Begriffe erläutert.

Messschläuche für medizinische Überwachungseinrichtungen sind flexible Schläuche, durch die im Rahmen der Überwachung oder Messung Fluide, insbesondere Gase (bspw. Druck-luft), durchtreten können. Bei Blutdruckmessungen erfolgt mittels dieser Messschläuche eine Druckmessung.

Die Kupplungsglieder können wieder lösbar zusammengesteckt werden. Dies bedeutet, dass die Verbindung durch einfaches axiales Zusammenstecken bzw. Wiederauseinanderziehen hergestellt und gelöst werden kann. Der Begriff "axiales Zusammenstecken" schließt nicht aus, dass beim Zusammenstecken oder Lösen ein leichtes Verkanten der Kupplungsglieder zueinander erfolgt. Zum Herstellen und Lösen der Verbindung ist es nicht erforderlich, Werkzeug zu verwenden, eine Schraub- oder Bajonettverbindung herzustellen bzw. zu lösen oder dergleichen.

Die Kupplungsglieder können gasdicht miteinander verbunden werden. "Gasdicht" bedeutet, dass die Verbindung hinreichend dicht für den vorgesehenen Anwendungszweck ist. Bei der Blutdruckmessung bspw. darf Luft nicht oder nur in einem solchen Maße entweichen, dass die Blutdruckmessung nicht oder nur unwesentlich beeinträchtigt wird. Jedes Kupplungsglied weist wenigstens einen Anschluss für einen Messschlauch auf. Bevorzugt sind ein oder zwei Schlauchanschlüsse vorgesehen (ein- oder zweilumige Ausführung). Ein Kupplungsglied mit zwei Schlauchanschlüssen dient der Verbindung mit sogenannten zweilumigen Messsystemen bzw. Blutdruckmanschetten. In der Praxis können zweilumige Messsysteme ohne Weiteres an einlumige Blutdruckmanschetten und umgekehrt angeschlossen werden. In diesem Fall weist ein erfindungsgemäßer Schlauchverbinder ein Kupplungsglied mit einem Anschluss für einen Messschlauch und ein dazu passendes zweites Kupplungsglied mit zwei Anschlüssen für Messschläuche auf.

Die Kupplungsglieder sind im Kupplungsbereich einstückig aus einem Kunststoff geformt. Die mechanische Verbindung und Gasdichtigkeit wird durch die einstückige Form der zueinander passendenden Kupplungsglieder bewirkt, ohne dass es separater Verbindungseinrichtungen, Dichtungselemente oder dergleichen bedarf. Einstückige Formung "im Kupplungsbereich" bedeutet, dass die Kupplungsglieder jedenfalls in den miteinander in Eingriff geratenden axialen Abschnitten (bezogen auf die Kupplungsachse) einstückig geformt sind. Ein geeigneter Kunststoff ist bspw. Polyoxymethylen.

Die Erfindung weist eine Reihe von in der Praxis wichtigen Vorteilen auf. Das Herstellen der Verbindung durch einfaches Zusammenstecken erlaubt eine wesentlich schnellere Handhabung als im Stand der Technik bekannte Systeme, bei denen bspw. eine Verschraubung hergestellt werden muss. Dies ist bspw. dann von Bedeutung, wenn ein Patient aus einem OP-Bereich auf eine Intensivstation gefahren wird. Die Blutdruckmessmanschette verbleibt in diesem Fall beim Patienten, im OP-Bereich wird durch einfaches Auseinanderziehen die Verbindung gelöst und im Intensivbereich wird der Schlauch der Blutdruckmanschette mit dem Schlauch des dort befindlichen Blutdruckmesssystems durch einfaches Zusammenstecken verbunden. Die erfindungsgemäß mögliche Ausgestaltung der Kupplungsglieder mit einem oder zwei (oder ggf. mehreren) Schlauchanschlüssen erlaubt es, an jeder Blutdruckmanschette und jedem Blutdruckmesssystem die Kupplungsglieder des erfindungsgemäßen Schlauchverbinders anzubringen, so dass jede Manschette mit jedem Blutdruckmesssystem zusammengesteckt werden kann. Dies ist in der Krankenhauspraxis ein erheblicher Vorteil, da gegenwärtig in der Praxis, bedingt insbesondere durch die in vielen Krankenhäusern übliche Verwendung von Ein- und Zweischlauchsystemen nebeneinander, eine Vielzahl verschiedener Manschettenanschlusssysteme zum Einsatz kommt und als Ersatz vorgehalten werden muss.

Die einstückige Formung der Kupplungsglieder aus Kunststoff im Kupplungsbereich erlaubt eine wesentlich verbesserte Krankenhaushygiene, da eine wesentlich einfachere und sicherere Reinigung, Desinfektion, Heiß- und/oder Dampfsterilisation der Schlauchverbinder möglich ist.

Die Kupplungsglieder greifen im Kupplungsbereich durch elastischen Formschluss ineinander. "Elastischer Formschluss" bedeutet, dass wenigstens eines der Kupplungsglieder im Kupplungsbereich eine gewisse Elastizität aufweist, die das Herstellen des Formschlusses erleichtert und ggf. im zusammengekuppelten Zustand eine Vorspannung im Kupplungsbereich aufbringt, die den mechanischen Zusammenhalt und die Dichtigkeit des Schlauchverbinders bewirkt bzw. verbessert.

Bevorzugt ist der Kupplungsbereich der Kupplungsglieder bezogen auf die Kupplungsachse rotationssymmetrisch. Dies bedeutet, dass die Kupplungsglieder in jeder Winkelstellung zusammengesteckt werden können und nicht vor dem Zusammenstecken in eine vorgegebene Winkellage zueinander orientiert werden müssen.

Vorzugsweise weist jedes Kupplungsglied eine Greifeinrichtung auf, die bspw. als Griffmulde ausgebildet sein kann. Die Griffmulden erlauben eine sichere Handhabung beim Zusammenstecken bzw. Lösen der Kupplungsglieder. Bevorzugte Außendurchmesser der Kupplungsglieder sind 0,5 bis 3 cm, weiter vorzugsweise 1 bis 2 cm. Es handelt sich um Durchmesser, die einerseits platzsparend sind, andererseits aber eine sichere manuelle Handhabung erlauben. Die axiale Länge der Kupplungsglieder (ohne angesetzte Schlauchanschlusstüllen) liegt bevorzugt zwischen 1,5 und 5 cm, weiter vorzugsweise zwischen 1,5 und 3 cm.

Die Kupplungsglieder können bevorzugt vollständig einstückig aus einem Kunststoff geformt sein. Diese einstückige Formung kann auch die Schlauchanschlusstüllen mit erfassen. Alternativ können die Schlauchanschlusstüllen aus einem anderen Material, vorzugsweise Metall, bestehen und mit der im Übrigen einstückig aus Kunststoff bestehenden Kupplung verbunden, insbesondere darin eingeformt sein.

Gegenstand der Erfindung ist ferner ein Set aus wenigstens drei Kupplungsgliedern, von denen jeweils zwei Kupplungsglieder zu einem erfindungsgemäßen Schlauchverbinder zusammensteckbar sind. Wenigstens zwei der drei Kupplungsglieder weisen einen Schlauchanschluss und wenigstens ein Kupplungsglied zwei Schlauchanschlüsse auf. Ein solches Set ermöglicht die Nachrüstung bzw. Umrüstung sowohl ein- als auch zweilumiger Systeme zur Blutdruckmessung mittels erfindungsgemäßen Schlauchverbindern.

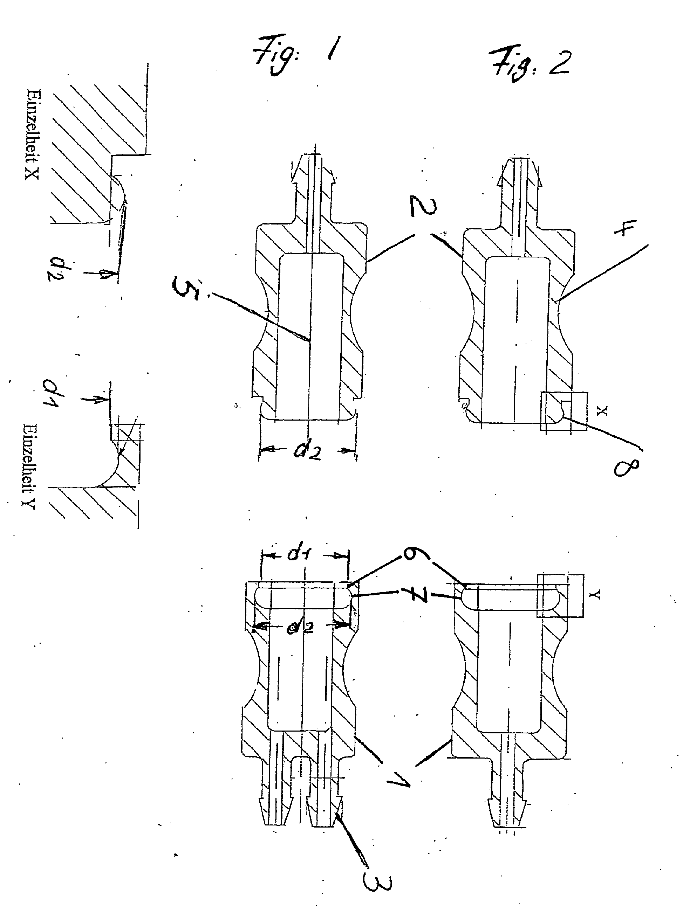

Eine Ausführungsform der Erfindung wird nachfolgend unter Bezugnahme auf die Zeichnung beschrieben. Die Figur zeigt erfindungsgemäße Schlauchverbinder schematisch in einem Querschnitt von der Seite. Fig. 1 zeigt eine Ausführungsform, bei der ein Kupplungselement ein Kupplungsglied zwei Schlauchanschlusstüllen aufweist, in Fig. 2 weisen beide Kupplungsglieder je ein Schlauchanschlussstück auf.

Die erfindungsgemäßen Schlauchverbinder weisen jeweils ein male-Kupplungsglied 2 und ein female-Kupplungsglied 1 auf. Schlauchanschlusstüllen 3 ermöglichen das Aufstecken der Messschläuche. Sie sind auf der dem Kupplungsbereich abgewandten Stirnseite der Kupplungsglieder 1 und 2 angeordnet. Griffmulden 4 erlauben eine leichtere Handhabung der Kupplungsglieder. Die Grundform der Kupplungsglieder im Kupplungsbereich ist zylindrisch bezogen auf die Kupplungsachse 5.

Das erste Kupplungsglied 1 (female) weist einen der Kupplungsseite zugewandten Eintrittsabschnitt 6 auf. Der lichte Durchmesser d₁ des Eintrittsabschnittes 6 beträgt im Ausführungsbeispiel 13 mm. Der Eintrittsabschnitt 6 ist im Wesentlichen als Hohlzylinder ausgebildet, die der Kupplungsseite zugewandte Stirnfläche des Eintrittsbereichs ist abgerundet, um das Einkuppeln eines zweiten Kupplungselements 2 (male) zu erleichtern.

Axial an den Eintrittsabschnitt 6 schließt sich ein radial nach außen erweiterter und eine größere radiale lichte Weite d₂ (im Ausführungsbeispiel 13,45 mm) aufweisender Halteabschnitt 7 an. Die gegenüber dem Eintrittsabschnitt 6 radial nach außen erweiterten Bereiche des Halteabschnitts 7 sind abgerundet ausgebildet und üben gegenüber einem entsprechend formschlüssig ausgebildeten Abschnitt des zweiten Kupplungsgliedes 2 eine Halte- und Dichtfunktion aus, wie nachstehend erläutert.

Das zweite Kupplungsglied 2 weist Kupplungsseitig einen Halteabschnitt 8 auf, dessen axiale und radiale Erstreckung im Wesentlichen komplementär zu dem Halteabschnitt 7 des ersten Kupplungsglieds 1 ist. Er kann daher im Wesentlichen formschlüssig in diesen Halteabschnitt 7 eingreifen. Sein Durchmesser d₂ entspricht dem Durchmesser des Halteabschnitts 7. Gegenüber dem Eintrittsabschnitt 6 des ersten Kupplungsgliedes 1 weist der Halteabschnitt 8 ein radiales Übermaß von etwa 0,6 mm (relativ betrachtet etwa 5 %) bezogen auf den Durchmesser auf. Beim Zusammenstecken oder Lösen der Kupplungsglieder kann der Halteabschnitt 8 unter elastischer Verformung des Eintrittsabschnitts 6 und ggf. auch des Halteabschnitts 8 durch diesen Eintrittsabschnitt 6 hindurchtreten und mit dem Halterabschnitt 7 des ersten Kupplungsgliedes in Eingriff geraten. Im zusammengesteckten Zustand wird der Kupplungsbereich des zweiten Kupplungselements 2 einschließlich des Haltebereichs 8 radial vom Kupplungsbereich des ersten Kupplungsgliedes 1 (dieser Kupplungsbereich umfasst den Haltebereich 7 und den Eintrittsbereich 6) umgriffen und die gasdichte Verbindung der beiden Kupplungsglieder wird durch Formschluss und ggf. eine elastische Vorspannung des Kunststoffmaterials des Kupplungsglieds hergestellt. Sofern die Schläuche mit Druck beaufschlagt werden, erhöht sich durch Anpressung der Halteabschnitt 7 und 8 gegeneinander die Dichtigkeit der Schlauchverbindung weiter. Die erfindungsgemäße Schlauchverbindung kann vielfach durch einfaches Zusammenstecken bzw. Auseinanderziehen der Kupplungsglieder hergestellt und wieder gelöst werden.

## Patentansprüche

1. Schlauchverbinder zum Verbinden von Messschläuchen für medizinische Überwachungseinrichtungen, der zwei Kupplungsglieder (1, 2) mit folgenden Merkmalen aufweist:
a) die Kupplungsglieder (1, 2) können gasdicht und wieder lösbar zusammengesteckt werden,
b) jedes Kupplungsglied (1, 2) weist wenigstens einen Anschluss (3) für einen Messschlauch auf,
c) die Kupplungsglieder (1, 2) sind im Kupplungsbereich einstückig aus einem Kunststoff geformt,
wobei das erste Kupplungsglied (1) im Kupplungsbereich einen der Kupplungsseite zugewandten im wesentlichen als Hohlzylinder ausgebildeten Eintrittsabschnitt (6) und axial daran anschließend einen gegenüber dem Eintrittsabschnitt (6) radial nach außen erweiterten und eine größere radiale lichte Weite aufweisenden Halteabschnitt (7) aufweist, dessen gegenüber dem Eintrittsabschnitt (6) radial nach außen erweiterten Bereiche abgerundet ausgebildet sind; und dass das zweite Kupplungsglied (2) kupplungsseitig einen Halteabschnitt (8) aufweist, der im wesentlichen formschlüssig in den Halteabschnitt (7) des ers-ten Kupplungsgliedes eingreifen kann und der gegenüber dem Eintrittsabschnitt (6) des ersten Kupplungsgliedes (1) ein radiales Übermaß aufweist, so dass er beim Zusammenstecken und Lösen der Kupplungsglieder (1, 2) unter elastischer Verformung eines oder beider Kupplungsglieder (1, 2) durch den Eintrittsabschnitt (6) hindurchtreten kann, wobei im zusammengesteckten Zustand der Kupplungsbereich des zweiten Kupplungsgliedes (2) radial vom Kupplungsbereich des ersten Kupplungsgliedes (1) umgriffen wird, wobei die Kupplungsglieder (1, 2) im Kupplungsbereich durch elastischen Formschluss ineinandergreifen und die gegenüber dem Eintrittsabschnitt (6) radial nach außen erweiterten Bereiche des Halteabschnitts (7) gegenüber dem Halteabschnitt (8) des zweiten Kupplungsgliedes (2) eine Halte- und Dichtfunktion ausübt.

2. Schlauchverbinder nach Anspruch 1, **dadurch gekennzeichnet, dass** der Kupplungsbereich der Kupplungsglieder (1, 2) bezogen auf eine Kupplungsachse (5) rotationssymmetrisch ist.

3. Schlauchverbinder nach Anspruch 1 oder 2, **dadurch gekennzeichnet, dass** jedes Kupplungsglied (1, 2) eine Greifeinrichtung (4) aufweist.

4. Schlauchverbinder nach Anspruch 3, **dadurch gekennzeichnet, dass** die Greifeinrichtungen als Griffmulden (4) ausgebildet sind.

5. Schlauchverbinder nach einem der Ansprüche 1 bis 4, **dadurch gekennzeichnet, dass** die Kupplungsglieder (1, 2) einen Außendurchmesser von 0,5 bis 3 cm, bevorzugt 1 bis 2 cm aufweisen.

6. Schlauchverbinder nach einem der Ansprüche 1 bis 5, **dadurch gekennzeichnet, dass** die Kupplungsglieder (1, 2) eine axiale Länge von 1,5 bis 5 cm, bevorzugt 1,5 bis 3 cm aufweist.

7. Schlauchverbinder nach einem der Ansprüche 1 bis 6, **dadurch gekennzeichnet, dass** die Kupplungsglieder (1, 2) vollständig einstückig aus einem Kunststoff geformt sind.

8. Schlauchverbinder nach einem der Ansprüche 1 bis 6, **dadurch gekennzeichnet, dass** die Kupplungsglieder (1, 2) jeweils ein oder zwei Schlauchanschlusstüllen (3) aufweisen.

9. Schlauchverbinder nach Anspruch 8, **dadurch gekennzeichnet, dass** die Kupplungsglieder (1, 2) bis auf die Schlauchanschlusstüllen (3), einstückig aus einem Kunststoff geformt sind.

10. Schlauchverbinder nach Anspruch 9, **dadurch gekennzeichnet, dass** die Schlauchanschlusstüllen (3) aus Metall bestehen.

11. Set aus wenigstens drei Kupplungsgliedern, von denen jeweils zwei Kupplungsglieder (1, 2) zu einem Schlauchverbinder nach einem der Ansprüche 1 bis 10 zusammensteckbar sind, **dadurch gekennzeichnet, dass** wenigstens zwei Kupplungsglieder (1, 2) einen Schlauchanschluss (3) und wenigstens ein Kupplungsglied (1, 2) zwei Schlauchanschlüsse (3) aufweist.

## Claims

1. Tube connector for connecting measuring tubes for medical monitoring devices, which comprises two coupling elements (1, 2) with the following features:
a) the coupling elements (1, 2) can be fitted together in a gastight and re-releasable manner,
b) each coupling element (1, 2) has at least one connection (3) for a measuring tube
c) in the coupling region, the coupling elements (1, 2) are formed in one piece from a plastic,
the first coupling element (1) having in the coupling region an entry portion (6), facing the coupling side and formed essentially as a hollow cylinder, and axially adjoining it, a holding portion (7), which is expanded radially outwards and has a greater clear radial width in comparison with the entry portion (6) and the regions of which that are expanded radially outwards in comparison with the entry portion (6) are formed such that they are rounded off; and the second coupling element (2) having on the coupling side a holding portion (8) which can essentially engage positively into the holding portion (7) of the first coupling element and is radially oversized in comparison with the entry portion (6) of the first coupling element (1), so that, when the coupling elements (1, 2) are fitted together and released, it can pass through the entry portion (6) while one or both of the coupling elements (1, 2) undergoes elastic deformation, the coupling region of the second coupling element (2) being gripped around radially by the coupling region of the first coupling element (1) in the fitted-together state, the coupling elements (1, 2) engaging in each other by elastic positive engagement in the coupling region and the regions of the holding portion (7) that are expanded radially outwards in comparison with the entry portion (6) exerting a holding and sealing function with respect to the holding portion (8) of the second coupling element (2).

2. Tube connector according to Claim 1, **characterized in that** the coupling region of the coupling elements (1, 2) is rotationally symmetrical with respect to a coupling axis (5).

3. Tube connector according to Claim 1 or 2, **characterized in that** each coupling element (1, 2) has a gripping device (4).

4. Tube connector according to Claim 3, **characterized in that** the gripping devices are formed as recessed grips (4).

5. Tube connector according to one of Claims 1 to 4, **characterized in that** the coupling elements (1, 2) have an outside diameter of 0.5 to 3 cm, with preference 1 to 2 cm.

6. Tube connector according to one of Claims 1 to 5, **characterized in that** the coupling elements (1, 2) have an axial length of 1.5 to 5 cm, with preference 1.5 to 3 cm.

7. Tube connector according to one of Claims 1 to 6, **characterized in that** the coupling elements (1, 2) are formed completely in one piece from a plastic.

8. Tube connector according to one of Claims 1 to 6, **characterized in that** the coupling elements (1, 2) respectively have one or two tube connecting sleeves (3).

9. Tube connector according to Claim 8, **characterized in that** the coupling elements (1, 2) are formed in one piece from a plastic with the exception of the tube connecting sleeves (3).

10. Tube connector according to Claim 9, **characterized in that** the tube connecting sleeves (3) consist of metal.

11. Set of at least three coupling elements, of which in each case two coupling elements (1, 2) can be fitted together to form a tube connector according to one of Claims 1 to 10, **characterized in that** at least two coupling elements (1, 2) have one tube connection (3) and at least one coupling element (1, 2) has two tube connections (3).

## Revendications

1. Raccord de tuyaux pour la connection de tuyaux de mesure destinés à des équipements médicaux de surveillance, qui comporte deux organes d'accouplement (1, 2) présentant les caractéristiques suivantes :
les organes d'accouplement (1, 2) peuvent être assemblés de manière étanche au gaz et de manière détachable,
chaque organe d'accouplement (1, 2) comporte au moins un raccord (3) pour un tuyau de mesure,
les organes d'accouplement (1, 2) sont formés, d'un seul tenant en une matière plastique, dans la zone d'accouplement ,
le premier organe d'accouplement (1) présentant dans la zone d'accouplement, une partie d'admission (6), conformée sensiblement en cylindre creux, tournée vers le côté d'accouplement et raccordée axialement à ladite partie, une partie de retenue (7) radialement élargie vers l'extérieur par rapport à la partie d'admission (6), présentant un diamètre intérieur radial supérieur, dont les zones élargies radialement vers l'extérieur par rapport à la partie d'admission (6) sont arrondies ; et le second organe d'accouplement (2) présentant côté accouplement, une partie de retenue (8) qui peut s'engager sensiblement par complémentarité de forme, dans la partie de retenue (7) du premier organe d'accouplement et comporte une surépaisseur radiale par rapport à la partie d'admission (6) de l'organe d'accouplement (1), de sorte que lors de l'emboîtement et du détachement des organes d'accouplement (1, 2), l'un ou les deux organes d'accouplement (1, 2) peuvent traverser la partie d'admission (6) par déformation élastique, à l'état emboîté la zone d'accouplement du second organe d'accouplement (2) étant entourée radialement par la zone d'accouplement du premier organe d'accouplement (1), les organes d'accouplement (1, 2) étant engagés l'un dans l'autre par complémentarité de forme dans la partie d'accouplement, et les zones radialement élargies de la partie de retenue (7) par rapport à la partie d'admission (6) exerçant une fonction de maintien et d'étanchéité par rapport à la partie de retenue (8) du second organe d'accouplement (2).

2. Raccord selon la revendication 1, **caractérisé en ce que** la zone d'accouplement des organes d'accouplement (1, 2) présente une symétrie de révolution par rapport à un axe d'accouplement (5).

3. Raccord selon l'une quelconque des revendications 1 ou 2, **caractérisé en ce que** chaque organe d'accouplement (1, 2) comporte un élément de préhension (4).

4. Raccord selon la revendication 3, **caractérisé en ce que** les éléments de préhension sont conformés en plages creuses (4).

5. Raccord selon l'une quelconque des revendications 1 à 4, **caractérisé en ce que** les organes d'accouplement (1, 2) présentent de préférence un diamètre de 0,5 à 3 cm, de préférence de 1 à 2 cm.

6. Raccord selon l'une quelconque des revendications 1 à 5, **caractérisé en ce que** les organes d'accouplement (1, 2) présentent une longueur axiale de 1,5 à 5 cm, de préférence 1,5 à 3 cm.

7. Raccord selon l'une quelconque des revendications 1 à 6, **caractérisé en ce que** les organes d'accouplement (1, 2) sont formés entièrement d'un seul tenant en une matière plastique,

8. Raccord selon l'une quelconque des revendications 1 à 6, **caractérisé en ce que** les organes d'accouplement (1, 2) présentent chacun un ou deux embouts porte-tuyau (3).

9. Raccord selon la revendication 8, **caractérisé en ce que** les organes d'accouplement (1, 2) sont formés d'un seul tenant en une matière plastique, à l'exception des embouts porte-tuyau (3).

10. Raccord selon la revendication 9, **caractérisé en ce que** les embouts porte-tuyau (3) sont constitués de métal.

11. Jeu de trois organes d'accouplement au moins, parmi lesquels respectivement deux organes d'accouplement (1, 2) peuvent être emboîtés pour former un raccord selon les revendications 1 à 10, **caractérisé en ce qu'**au moins deux organes d'accouplement (1, 2) comportent un raccord pour tuyau (3) et qu'au moins un organe d'accouplement (1, 2) comporte deux raccords pour tuyau (3).
